# EUROPEAN PATENT SPECIFICATION

(11) **EP 2 494 929 B1**
(45) Date of publication and mention of the grant of the patent: **11.04.2018**
(21) Application number: 10826957.2
(22) Date of filing: 01.07.2010
(51) Int. Cl.: A61B 17/04, A61B 17/06, A61B 17/062, A61B 17/29

(54) **SURGICAL SUTURING APPARATUS**
VORRICHTUNG FÜR CHIRURGISCHES NÄHEN
APPAREIL À SUTURES CHIRURGICALES

(30) Priority: 26.10.2009 KR 20090101837
(43) Date of publication of application: 05.09.2012
(73) Proprietor: National Cancer Center, Ilsandong-gu Goyang-si, Gyeonggi-do 410-769 (KR)
(72) Inventor: CHO, Seong-Yeon, Goyang-si Gyeonggi-do 410-769 (KR); PARK, Sang-Jae, Goyang-si Gyeonggi-do 410-769 (KR); KIM, Seoung-Hoon, Goyang-si Gyeonggi-do 410-769 (KR); HAN, Sung-Sik, Goyang-si Gyeonggi-do 410-769 (KR); KIM, Kwang-Gi, Goyang-si Gyeonggi-do 410-769 (KR); KIM, Soo-Hyun, Goyang-si Gyeonggi-do 410-769 (KR)
(74) Representative: Zimmermann, Tankred Klaus
(86) International application number: PCT/KR2010/004283
(87) International publication number: WO 2011/052872

(56) References cited:
- WO-A1-93/16644
- JP-A- 8 215 199
- JP-A- 2003 052 702
- JP-A- 2003 052 702
- KR-A- 20050 033 979
- US-A- 5 603 718
- US-A1- 2004 260 314
- US-A1- 2007 270 887

## Description

### [Technical Field]

The present invention relates to a surgical instrument, and more particularly to a surgical suture instrument used to suture tissues of a patient in the event of a laparotomy and laparoscopic surgery.

### [Background Art]

In general, suture needles used for surgical operations are curved needles. Thus, to suture bodily tissues with minimum damage, the suture needle should be held at a right angle by a needle holder, unlike a straight needle, and then the needle holder should be turned.

However, when such a needle holder is used, suturing is difficult in the event of a laparotomy and laparoscopic surgery in which a field of view is narrow and undesired damage to a tissue is frequently caused by the suture needle. Further, in the event of laparoscopic surgery, the needle holder is adapted so that a rotary shaft thereof is fixed to a laparoscopic port having a preset position. Thus, a suture plane parallel to the needle holder (i.e. perpendicular to the suture needle) is sutured with relative ease, but a suture plane perpendicular to the needle holder (i.e. parallel to the suture needle) is difficult to suture. For this reason, unnecessary damage to a tissue frequently happens. In particular, for the suture in the laparoscopic surgery, a suture needle is inserted into an abdominal cavity first, and forceps are inserted into a laparoscopic port to seize the suture needle. Subsequently, a needle holder is inserted into another laparoscopic port, and holds the suture needle at a right angle. Thus, difficulties are caused in terms of manipulation, and a long operation time is required. Further, since instruments for the laparoscopic surgery including such a needle holder have a long length of about 50 cm, an operating surgeon has difficulty performing elaborate suturing based on accurate reflection of hand movement such as rotation. Thus, the suture needle is frequently inserted into an undesired suture spot, and tissues are frequently damaged in the process of rotating the needle holder holding the suture needle to pierce the tissues with the suture needle.

US 2007/270887 A1 describes a circular needle pusher having a body, a handle, a needle pushing trigger connecting piece, a needle pushing trigger, a spindle, a needle capturing trigger, a rotation point, a lock mechanism, a needle holder control arm, a cap, a cap rotation point, a needle holder jaws, a fixing arm, a pusher arm, a needle slot, a pusher arm slot, a slot rotation point, a slot spindle, a needle and a needle canal.

JP 2003 052702 A describes a suturing apparatus simplifying the use of a suturing thread and a suturing needle. On a top side of a supporting tube for holding a reciprocative member to reciprocate, a ring-shaped suturing needle guide is provided with a notched part to insert a viable tissue as a part to be sutured, in the middle. Inside the suturing needle guide, an arcuate suturing needle is housed to be rotatable. A slider is housed in one half ring area of the suturing needle guide and a slider is housed in the other half ring area. The sliders are connected with the reciprocative member through first links and connected with a link fixing part through second links.

US 2004/260314 A1 describes a needle passer instrument for use in minimally invasive surgical procedures, including arthroscopy. The instrument has upper and lower jaws for engaging tissue and a handle. A removable needle engaging cartridge is mounted to the upper jaw. A surgical needle with attached suture is mounted in a needle passage in the lower jaw. A needle actuation rod engages the surgical needle and pushes the needle through tissue contained between the jaws. The needle is engaged by the cartridge, and the needle may be cut away from the suture.

### [Disclosure]

### [Technical Problem]

The present invention relates to a surgical suture instrument as claimed hereafter.

Preferred embodiments are set forth in the dependent claims.

Accordingly, the present invention has been made keeping in mind the above problems occurring in the related art, and an object of the present invention is to provide a surgical suture instrument that is structurally improved so as to minimize undesired damage to tissues caused by a suture needle in the event of a laparotomy and laparoscopic surgery and to enable elaborate safe suturing.

The objects of the present invention are not limited to the object described above, and the other objects not stated above will be clearly understood from the following description by those skilled in the art.

### [Technical Solution]

To achieve the object, according to an aspect of the present invention, there is provided a surgical suture instrument including: a shank; a needle actuator installed at one side of the shank, into which a suture needle is inserted, squeezing a desired suture spot through primary rotation, and pushing the suture needle by secondary rotation to pass through the suture spot to suture the suture spot; and a driver installed at the other side of the shank and sequentially driving the squeezing and suturing operations of the needle actuator. The shank has a wire hole in which first and second wires are held. The needle actuator includes an inlet guide rotatably coupled to one side of a leading end of the shank, connected to the first wire, and guiding insertion of the suture needle; a needle pusher rotatably coupled to the other side of the leading end of the shank, connected to the second wire, and rotating toward the inlet guide to push the suture needle such that the suture needle inserted into the inlet guide passes through the suture spot to suture the suture spot; and an outlet guide fixed to the leading end of the shank, engaging with the inlet guide to squeeze the suture spot, and guiding passage of the suture needle passing through the suture spot.

The shank may have a tubular shape.

In addition, an inlet guide and a needle pusher, which are to be described later, may be rotatably connected to a leading end of the shank, and a first linkage to which the outlet guide is fixedly connected may be provided at the leading end.

Here, a long hole corresponding to a radius of rotation of the needle pusher may be formed in the first linkage such that the needle pusher is rotated toward the inlet guide to pass therethrough.

In addition, a second linkage to which a handle (described later) is rotatably connected may be provided at a trailing end of the shank.

The inlet guide may include an inlet link member rotatably linked to the first linkage about an axis perpendicular to a longitudinal direction of the shank; and an inlet guide member fixed to the inlet link member, having an arc shape corresponding to the curved suture needle, and having an inlet hole into which the suture needle is inserted.

Here, wherein the inlet link member may have a first wire connecting hole, to which the first wire is connected, formed at an opposite area of the inlet guide member with respect to the first linkage.

The needle pusher may include a push link member rotatably linked to the first linkage about the same axis as the inlet link member; and a push guide member fixed to the push link member, having an arc shape corresponding to the inlet guide member, and rotated to be inserted into the inlet guide member and pushing the suture needle inserted into the inlet guide member.

Here, the push link member may have a second wire connecting hole, to which the second wire is connected, formed at an opposite area of the push guide member with respect to the first linkage.

In addition, an outer diameter of the push guide member may be at least smaller than a diameter of the suture needle, and a length of the push guide member may be equal to a length of the inlet guide member or at least larger than a length of the inlet guide member.

The outlet guide may include an outlet fixture member fixed to the first linkage by a fixing shaft having an angle perpendicular to a longitudinal direction of the shank; and an outlet guide member fixed to the outlet fixture member, having an arc shape corresponding to the inlet guide member, and having an outlet hole through which the suture needle passing through the suture spot passes.

Here, the inlet guide member and the outlet guide member may have inner diameters corresponding to each other, and larger than a diameter of the suture needle.

In addition, the inlet guide member and the outlet guide member may have lengths corresponding to each other, and at least larger than a length of the suture needle.

Further, the inlet guide member, the push guide member and the outlet guide member may be circumferentially disposed along a concentric circle having the same radius about the first linkage.

The driver may be installed at a trailing end of the shank, and provide tension to the first wire and the second wire to sequentially rotate the inlet guide and the needle pusher.

The driver may include a handle coupled to the trailing end of the shank and gripped by a user; a first operation unit pivotally coupled to one side of the handle, connected to the first wire, and providing tension to the first wire by compression of the user to rotate the inlet guide toward the outlet guide; and a second operation unit pivotally coupled to the other side of the handle, connected to the second wire, and providing tension to the second wire by compression of the user to rotate the needle pusher toward the inlet guide.

In addition, a surgical suture instrument in accordance with the present invention includes a shank in which first and second wires are held; an inlet guide rotatably coupled to one side of a leading end of the shank, connected to the first wire, and guiding insertion of a suture needle; a needle pusher rotatably coupled to the other side of the leading end of the shank, connected to the second wire, and rotating toward the inlet guide to push the suture needle such that the suture needle inserted into the inlet guide passes through the suture spot to suture the suture spot; an outlet guide fixed to the leading end of the shank, engaging with the inlet guide to squeeze the suture spot, and guiding passage of the suture needle passing through the suture spot; and a driver installed at a trailing end of the shank, and providing tension to the first wire and the second wire to sequentially rotate the inlet guide and the needle pusher.

Further, a surgical suture instrument in accordance with the present invention includes a shank; an inlet guide rotatably coupled to one side of a leading end of the shank and guiding insertion of a suture needle; a needle pusher rotatably coupled to the other side of the leading end of the shank, and rotating toward the inlet guide to push the suture needle such that the suture needle inserted into the inlet guide passes through a suture spot; an outlet guide fixed to the leading end of the shank, and guiding passage of the suture needle passing through the suture spot; and a driver installed at a trailing end of the shank, and sequentially rotating the inlet guide and the needle pusher.

The details of other exemplary embodiments are included in the detailed description and the drawings.

### [Advantageous Effects]

The surgical suture instrument of the present invention can squeeze a desired suture spot through primary rotation after insertion of the suture needle, and push the suture needle to pass through the suture spot by secondary rotation to enable elaborate suturing with respect to the suture spot, minimizing undesired damage to the tissue by the suture needle.

In addition, as the squeezing and suturing operations of the suture spot are performed through rotation, safe suturing can be performed regardless of a direction of a suture plane.

Further, since an operation of the suture needle is simple, an operation time can be reduced.

Furthermore, since the suturing using a single laparoscopic port is possible, an incision surface of the body can be minimized.

The effects of the present invention are not limited to the effects described above, and the other effects not stated in the above will be clearly understood by those skilled in the art from the following description.

### [Brief Description of the Drawings]

FIG. 1 is a perspective view of a surgical suture instrument in accordance with an exemplary embodiment of the present invention;
FIG. 2 is a side view of the surgical suture instrument shown in FIG. 1;
FIG. 3 is a perspective view of a shank shown in FIG. 1;
FIG. 4 is an enlarged view of part A shown in FIG. 1;
FIG. 5 is an exploded perspective view of FIG. 4;
FIG. 6 is a schematic view of the part A shown in FIG. 1;
FIG. 7 is an enlarged view of part B shown in FIG. 1;
FIG. 8 is an exploded perspective view of FIG. 7;
FIGS. 9 to 12 are example views for sequentially explaining operations of the surgical suture instrument of the present invention;
FIG. 13 is a perspective view of a needle actuator in accordance with another exemplary embodiment of the present invention;
FIG. 14 is a side view of FIG. 13;
FIG. 15 is a plan view of FIG. 13;
FIG. 16 is an enlarged view of part C shown in FIG. 15;
FIG. 17 is a perspective view showing a rotational movement structure of a needle pusher with respect to a fixing pin;
FIG. 18 is a cross-sectional view taken along line X-X of FIG. 17;
FIG. 19 is an example view for explaining a first operation state of the needle actuator in accordance with another exemplary embodiment of the present invention;
FIG. 20 is a plan view of FIG. 19;
FIG. 21 is an enlarged view of part D shown in FIG. 20;
FIG. 22 is an example view for explaining a second operation state of the needle actuator;
FIG. 23 is a plan view of FIG. 22;
FIG. 24 is an enlarged view of part E shown in FIG. 23;
FIG. 25 is an example view for explaining a third operation state of the needle actuator;
FIG. 26 is a plan view of FIG. 25; and
FIG. 27 is an enlarged view of part F shown in FIG. 26.

### [Modes for Carrying out the Invention]

Advantages and features of the present invention and methods for achieving them will be made clear from exemplary embodiments described below in detail with reference to the accompanying drawings. However, the present invention is not limited to exemplary embodiments described herein and will be implemented in various forms. The exemplary embodiments are provided by way of example only so that a person of ordinary skill in the art can fully understand the disclosures of the present invention and the scope of the present invention. Therefore, the present invention will be defined only by the scope of the appended claims. Like reference numerals designate like components throughout the specification.

A surgical suture instrument according to an exemplary embodiment of the present invention will be described below with reference to the accompanying drawings. For reference, the detailed descriptions of known function and constructions unnecessarily obscuring the subject matter of the present invention will be avoided hereinafter.

Prior to the description of the present invention, a leading end of the surgical suture instrument can be defined as a part directed to a suture spot of bodily tissues, and a trailing end of the surgical suture instrument as a part opposite to the suture spot.

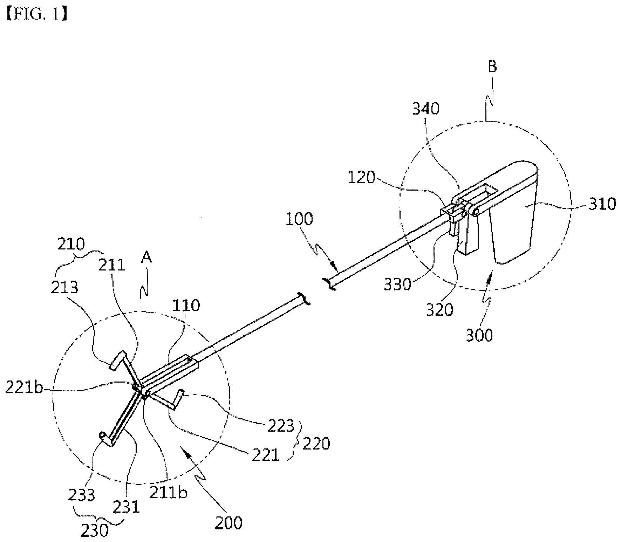

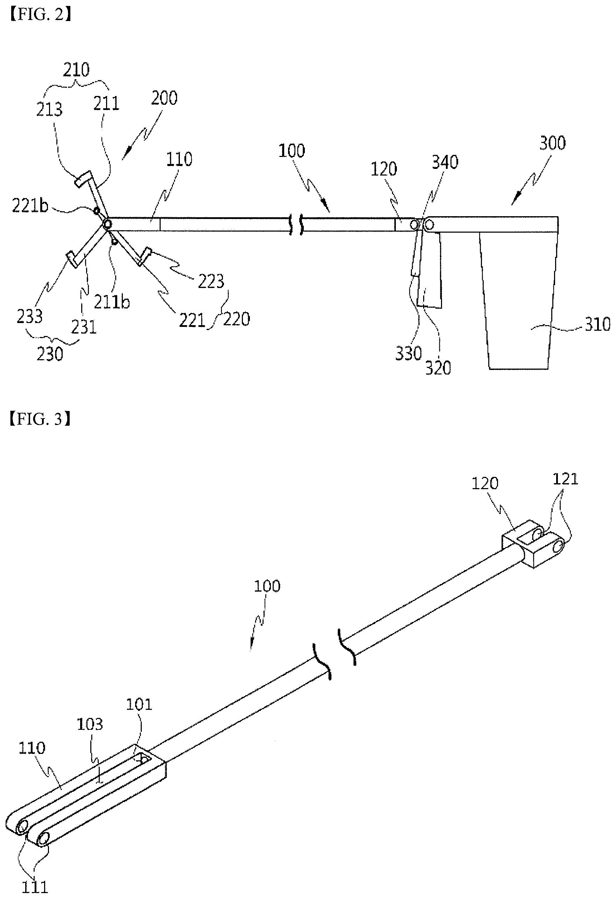

FIG. 1 is a perspective view showing a surgical suture instrument according to an exemplary embodiment of the present invention, and FIG. 2 is a side view of the surgical suture instrument shown in FIG. 1.

As shown in FIGS. 1 and 2, the surgical suture instrument according to an exemplary embodiment of the present invention includes a shank 100, a needle actuator 200, and a driver 300.

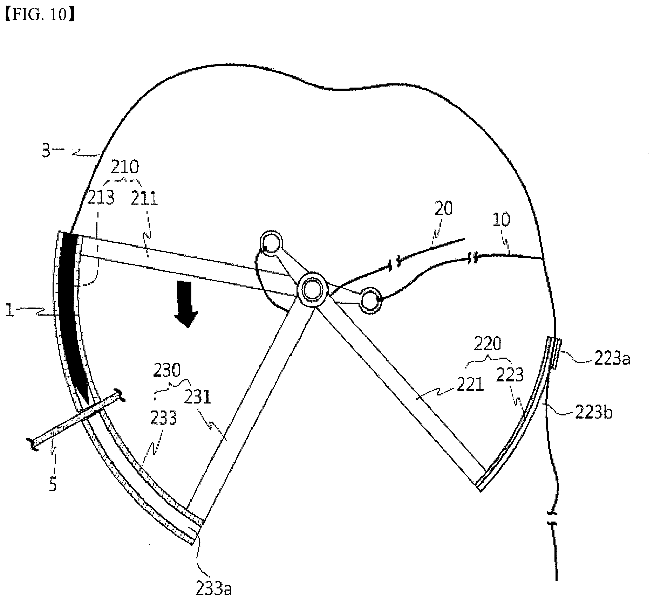

Here, the needle actuator 200 may be provided on one side of the shank 100, for instance a leading end of the shank 100. The needle actuator 200 serves to receive a suture needle 1 (see FIG. 1), squeeze a desired suture spot 5 (see FIG. 10) of bodily tissues by means of primary rotation, and to force the suture needle 1 to pierce and suture the suture spot 5 by means of secondary rotation. The suture needle 1 used in the surgical suture instrument of the present invention is preferably designed so that a tip thereof is made of a rigid material such as metal and so that a body other than the tip is made of a flexible material so as to be able to be inserted into arc-shaped inlet and outlet guide members 213 and 233, which will be described below, and smoothly move along a curved surface. For example, the material for the suture needle 1 may be the same material as a guide wire used when a central venous catheter is inserted. In the present embodiment, a configuration in which the surgical suture instrument uses a curved suture needle, i.e. a curved needle, is described by way of example. However, the surgical suture instrument may use a linear suture needle, i.e. a straight needle, which is formed of a flexible material.

Further, the driver 300 may be the other side of the shank 100, for instance a trailing end of the shank 100. The driver 300 sequentially drives squeezing and suturing motions of the needle actuator 200.

FIG. 3 is a perspective view of the shank shown in FIG. 1.

As shown in FIG. 3, the shank 100 is formed in a tubular shape, and connects between the needle actuator 200 and the driver 300. In the present embodiment, a configuration in which the shank 100 is formed in a cylindrical rod shape is illustrated. The shank is not limited to the cylindrical rod shape, but it may have a polygonal rod shape such as a quadrilateral rod shape or a hexagonal rod shape.

The shank 100 is provided therein with a wire hole 101 in a lengthwise direction so as to be able to hold first and second wires 10 and 20, which will be described below.

The leading end of the shank 100 is provided with a first linkage 110 to which an inlet guide 210 and a needle pusher 220, which will be described below, are rotatably connected, and to which an outlet guide 230, which will be described below, is fixedly connected. Here, the first linkage 110 is formed in an approximate U shape so as to be open to the front of the leading end of the shank 100, and is provided with fixing holes 111, into which a fixing pin 113 can be inserted so as to fix the outlet guide 230 to the first linkage 110, at an angle that is perpendicular to the lengthwise direction of the shank 100.

The first linkage 110 is provided with a long hole 103 that corresponds to a radius of rotation of the needle pusher 220 so that the needle pusher 220 can pass through the long hole 103 when rotated to the inlet guide 210. Here, the first linkage 110 may be provided with wire holes (not shown) at opposite sides thereof such that the first and second wires 10 and 20 can be inserted into the respective wire holes.

Further, the trailing end of the shank 100 is provided with a second linkage 120 to which a handle 310, which will be described below, is rotatably connected. Here, the second linkage 120 is formed is formed in an approximate U shape so as to be open to the rear of the trailing end of the shank 100, and is provided with hinge holes 121, into which a hinge shaft 341 of a hinge 340 coupled with the handle 310 can be inserted, at an angle that is perpendicular to the lengthwise direction of the shank 100.

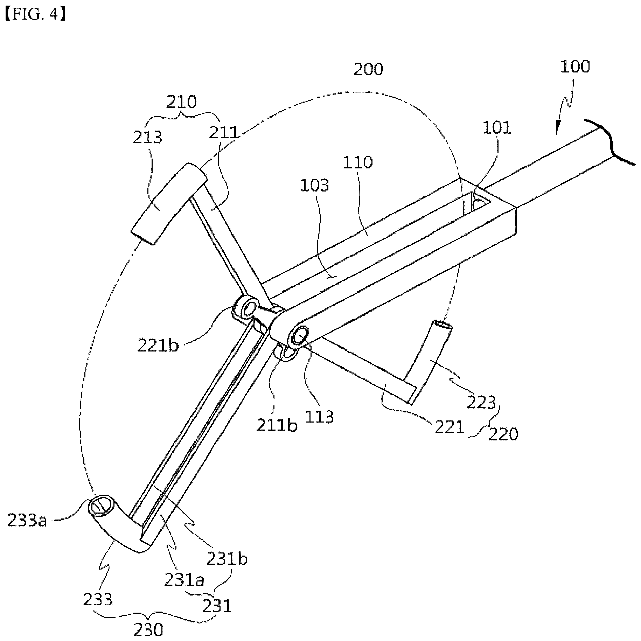

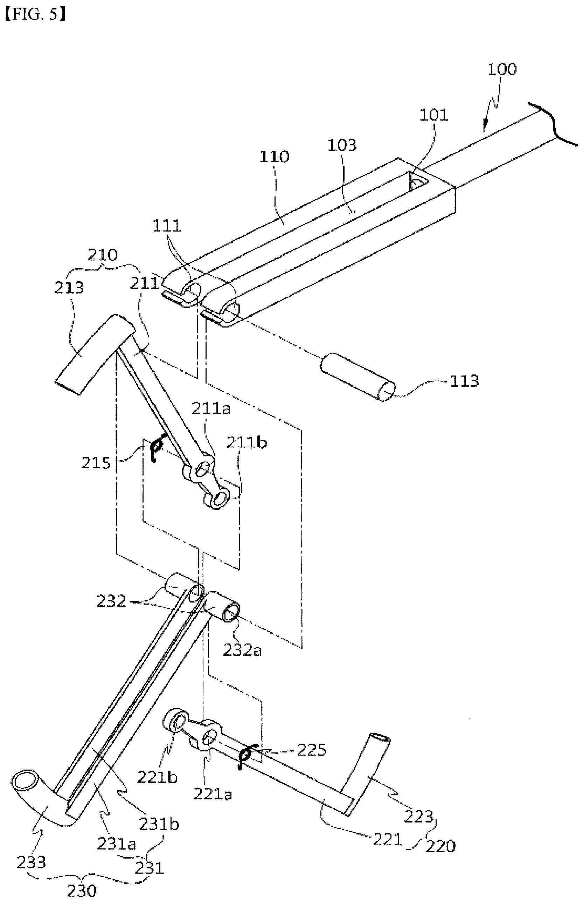

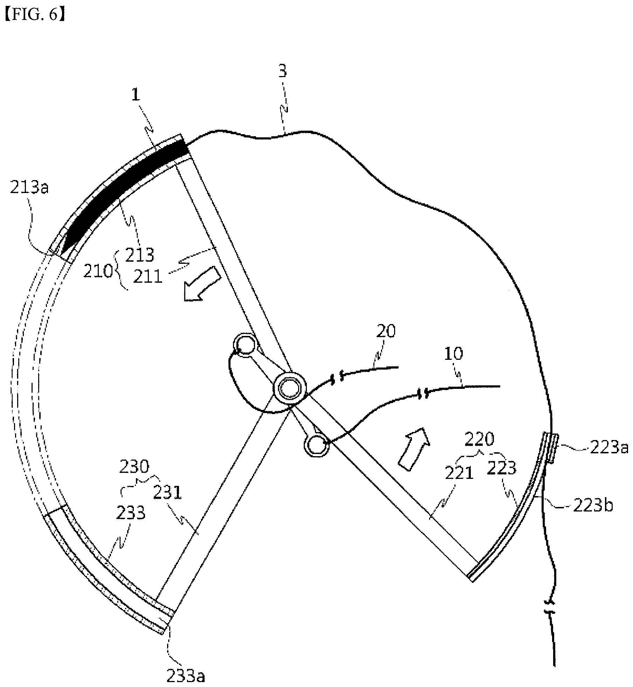

FIG. 4 is an enlarged view of the needle actuator (part A) shown in FIG. 1. FIG. 5 is an exploded perspective view of FIG. 4. FIG. 6 is a schematic configuration view of the part A shown in FIG. 1.

As shown in FIGS. 4 to 6, the needle actuator 200 includes the inlet guide 210, the needle pusher 220, and the outlet guide 230.

The inlet guide 210 is rotatably coupled at one side of the leading end of the shank 100, is connected with the first wire 10, and functions to guide insertion of the suture needle 1.

The inlet guide 210 includes an inlet link member 211 that is coupled so as to be able to rotate toward the outlet guide 230 above the shank 100 when the fixing pin 113, which is inserted into the fixing holes 111 of the first linkage 110, is fitted into a link connecting hole 211a, and the inlet guide member 213 which is fixed to an end of the inlet link member 211 and through which an inlet hole 213a passes for the insertion of the suture needle 1.

Here, the inlet link member 211 is provided with a first wire connecting hole 211b to which the first wire 10 is connected at the opposite side to the inlet guide member 213 with respect to the link connecting hole 211a connected with the first linkage 110. In the present embodiment, the inlet link member 211 is configured in a single rod shape by way of example. However, the inlet link member 211 is not limited to the single rod shape. Thus, the inlet link member 211 may be configured to be manufactured in a shape of parallel bars consisting of a pair of members like an outlet fixture member 231, which will be described below, and rotatably inserted between the parallel bars of the outlet fixture member 231. In this case, a push link member 221 having the shape of a single rod is inserted between the parallel bars of the inlet link member 211 so as to enable free rotation.

The inlet guide member 213 may be formed in the shape of a rod whose cross section is circular, or in an arc shape so as to correspond to the curved suture needle 1.

Further, an inlet hole 213a of the inlet guide member 213 has an inner diameter that corresponds to an outer diameter of the suture needle 1, an outer diameter of a push guide member as will be described below, and an inner diameter of the outlet guide member 233. In detail, preferably, the inner diameter of the inlet hole 213a of the inlet guide member 213 is slightly greater than the outer diameter of the suture needle 1 such that the suture needle 1 can be smoothly inserted into the inlet guide member 213, and is sufficiently greater than the outer diameter of the push guide member 223.

In addition, the inlet guide member 213 has a length so as to correspond to a length of the suture needle 1. In detail, preferably, the length of the inlet guide member 213 is preferably at least longer than that of the suture needle 1 such that the tip of the suture needle 1 is not exposed beyond the inlet guide member 213.

The needle pusher 220 is rotatably coupled at the other side of the leading end of the shank 100, is connected with the second wire 20, and rotates toward the inlet guide 210 to push the suture needle 1 such that the suture needle 1 inserted into the inlet guide 210 passes through and sutures the suture spot 5.

The needle pusher 220 includes a push link member 221 that is coupled to the first linkage 110 so as to be able to rotate toward the inlet guide 210 below the shank 100 with the fixing pin 113 fitted into a link connecting hole 221a so as to have the same axis as the inlet link member 211, and a push guide member 223 that is fixed to an end of the push link member 221, rotates toward the inlet guide member 213, is inserted into the inlet guide member 213, and pushes the suture needle 1 inserted into the inlet guide member 213.

Here, the push link member 221 is provided with a second wire connecting hole 221b to which the second wire 20 is connected at the opposite side to the push guide member 223 with respect to the link connecting hole 221a connected with the first linkage 110. In the present embodiment, the inlet link member 211 is configured in a single rod shape by way of example. However, the inlet link member 211 is not limited to the single rod shape. Thus, the inlet link member 211 may be configured to be manufactured in a shape of parallel bars consisting of a pair of members like an outlet fixture member 231, which will be described below, and rotatably inserted between the parallel bars of the outlet fixture member 231. In this case, a push link member 221 having the shape of a single rod is inserted between the parallel bars of the inlet link member 211 so as to enable free rotation.

The push guide member 223 has an arc shape corresponding to the inlet guide member 213 so as to be able to be inserted into the arc-shaped the inlet guide member 213.

Further, the outer diameter of the push guide member 223 is preferably formed so as to be sufficiently smaller than the inner diameter of the inlet guide member 213. For example, the push guide member 223 is formed in a cylindrical shape so as to have at least a smaller diameter than a rear end of the suture needle 1, and then is formed in a semi-cylindrical shape by cutting the remaining part other than a cylindrical portion 223a of a front end thereof in half. Thereby, the suture goes through the cylindrical portion 223a of the front end of the push guide member 223, and comes out of an open space of the semi-cylindrical portion 223b. Thus, when the push guide member 223 is inserted into the inlet guide member 213 and pushes the suture needle 1, the suture 3 connected to the suture needle 1 can smoothly enter the inlet guide member 213 without snapping off.

Further, the push guide member 223 has a length corresponding to the length of the inlet guide member 213. In detail, preferably, the push guide member 223 has the same length as the inlet guide member 213, or is at least longer than the inlet guide member 213.

The outlet guide 230 is fixed to the leading end of the shank 100, faces the inlet guide 210 to squeeze the suture spot 5, and guides the suture needle 1 penetrating the suture spot 5.

The outlet guide 230 includes an outlet fixing member 231, fixing shafts 232 of which are fixedly fitted into the fixing holes 111 of the first linkage 110 and which is disposed ahead in a rotational direction of the inlet guide 210 such that the suture spot 5 is located between the inlet guide 210 and the outlet guide 230, and an outlet guide member 233 that is fixed to an end of the outlet fixing member 231 and has an outlet hole 233a through which the suture needle 1 penetrating the suture spot 5 passes.

Here, the outlet fixing member 231 is made up of a pair of fixing members 231a and 231b, and the fixing shafts 232 protrude outwardly from the respective fixing members 231a and 231b at a right angle, and are fixedly fitted into the fixing holes 111 of the U-shaped first linkage 110. In this case, the inlet link member 211 and the push link member 221 are rotatably coupled by the fixing pin 113 fitted into shaft holes 232a formed in the fixing shafts 232 with the respective link connecting holes 211a and 221a inserted in a space between the fixing shafts 232.

The outlet guide member 233 may have a rod shape with a circular cross-section, and an arc shape corresponding to the curved suture needle 1.

In addition, the outlet hole 233a of the outlet guide member 233 may have an inner diameter corresponding to an inner diameter of the inlet guide member 213.

Further, the outlet guide member 233 has a length corresponding to a length of the inlet guide member 213. More specifically, the length of the outlet guide member 233 may be at least larger than that of the suture needle 1 such that the tip of the suture needle 1 is not exposed to the outside of the outlet guide member 233.

Furthermore, the inlet guide member 213, the push guide member 223 and the outlet guide member 233 may be circumferentially disposed along a concentric circle having the same radius about the first linkage 110.

A first recovering spring 215 may be installed to provide a rotational recovering force to the inlet guide 210 such that the inlet guide 210 engaged with the outlet guide 230 and squeezing the suture spot 5 is rotated in an opposite direction of the outlet guide 230 to return to the original position, when compression against a first operation unit 320 (described later) is released. Here, the first recovering spring 215 may be a torsion spring fitted onto an outer circumference of the fixing pin 113 of the first linkage 110 and having one end and the other end fixed to the inlet link member 211 and the outlet fixture member 231, respectively.

In addition, a second recovering spring 225 may be installed to provide a rotational recovering force to the needle pusher 220 such that the needle pusher inserted into the inlet guide 210 and pressing the suture needle 1 is rotated in an opposite direction of the inlet guide 210 to return to the original position, when compression against a second operation unit 330 (to be described below) is released. Here, the second recovering spring 225 may be a torsion spring fitted onto an outer circumference of the fixing pin 113 of the first linkage 110 and having one end and the other end fixed to the push link member 221 and the outlet fixture member 231, respectively.

Here, the structure of the torsion spring is well known in the art and will be apparent to those skilled in the art, and thus, detailed description thereof will be omitted.

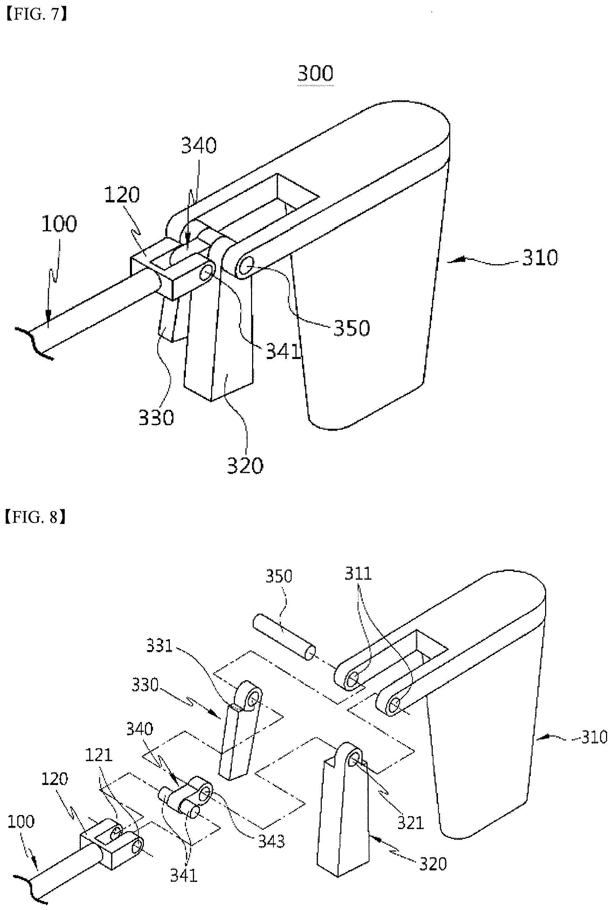

FIG. 7 is an enlarged perspective view of the driver (part B) shown in FIG. 1, and FIG. 8 is an exploded perspective view of FIG. 7.

As shown in FIGS. 7 and 8, the driver 300 is installed at the trailing end of the shank 100, and functions to sequentially rotate the inlet guide 210 and the needle pusher 220 to squeeze and suture the suture spot 5. That is, the driver 300 can provide tension to the first wire 10 connected to the inlet guide 210 and the second wire 20 connected to the needle pusher 220 to sequentially rotate the inlet guide 210 and the needle pusher 220.

The driver 300 may include the handle 310, the first operation unit 320 and the second operation unit 330.

The handle 310 has a shape that can be gripped by a user, and is coupled to the trailing end of the shank 100. For example, the handle 310 may be rotatably coupled to the second linkage 120 of the shank 100 by the hinge 340. Here, the hinge 340 can be connected to the shank 100 by inserting the hinge shaft 341 formed at one side into the hinge hole 121 of the second linkage 120, and connected to the handle 310 by inserting a coupling pin 350 into a hinge hole 343 formed at the other side. In addition, the handle 310 may have a pin hole 311, into which the coupling pin 350 is inserted, such that the hinge 340, the first operation unit 320 and the second operation unit 330 can be pivotally coupled thereto.

The first operation unit 320 may be pivotally coupled to one side of the handle 310 by the coupling pin 350. Here, a pin hole 321 is formed at an upper end of the first operation unit 320 such that the coupling pin 350 can be inserted.

In addition, the first operation unit 320 is connected to the first wire 10, and provides tension to the first wire 10 by compression of the user to rotate the inlet guide 210 toward the outlet guide 230.

The second operation unit 330 may be pivotally coupled to the other side of the handle 310 by the coupling pin 350. Here, a pin hole 331 is formed at an upper end of the second operation unit 330 such that the coupling pin 350 can be inserted.

Further, the second operation unit 330 is connected to the second wire 20, and provides tension to the second wire 20 by compression of the user to rotate the needle pusher 220 toward the inlet guide 210.

In the embodiment, while the first operation unit 320 and the second operation unit 330 are exemplarily described as having a configuration with a main trigger and an auxiliary trigger of a general gun, they are not limited thereto but may include various configurations.

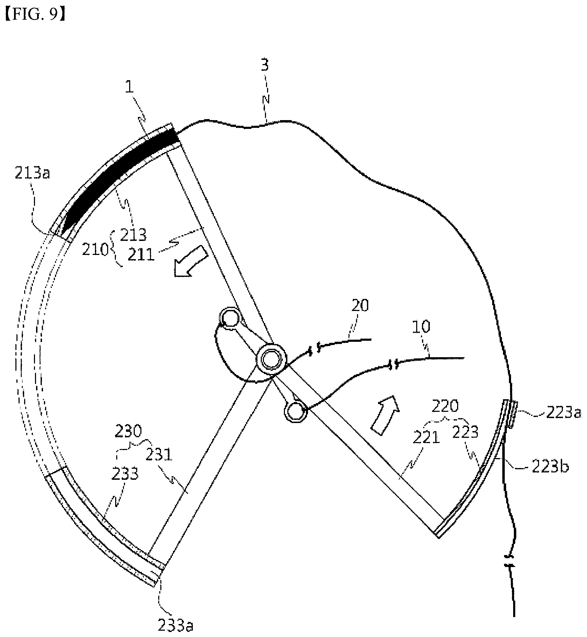

Hereinafter, an operation of the surgical suture instrument in accordance with an exemplary embodiment of the present invention will be described in detail with reference to FIGS. 9 to 12.

FIG 9 is a view showing a state in which the suture needle of the needle actuator of the surgical suture instrument is inserted.

As shown in FIG. 9, the suture needle 1 is fully inserted into the inlet hole 213a of the inlet guide member 213. Here, a suture wire 3 is connected to the trailing end of the suture needle 1. Since the inlet guide member 213 has an arc shape corresponding to the curved suture needle 1, the curved suture needle 1 can be easily inserted into the inlet guide member 213.

FIG. 10 is an example view for explaining the squeezing of the suture spot according to the first operation of the surgical suture instrument of the present invention.

As shown in FIG. 10, in a state in which the suture needle 1 is inserted into the inlet guide member 213, the desired suture spot 5 of the tissue is positioned between the inlet guide member 213 and the outlet guide member 233. Then, when the first operation unit 320 of the driver 300 is pulled by a finger, the first operation unit 320 is compressed and the first wire 10 pulls the inlet link member 211, and thus, the inlet guide member 213 into which the suture needle 1 is inserted is rotated toward the outlet guide 230 about the first linkage 110. Here, the inlet guide member 213 is engaged with the outlet guide member 233 to squeeze the suture spot 5.

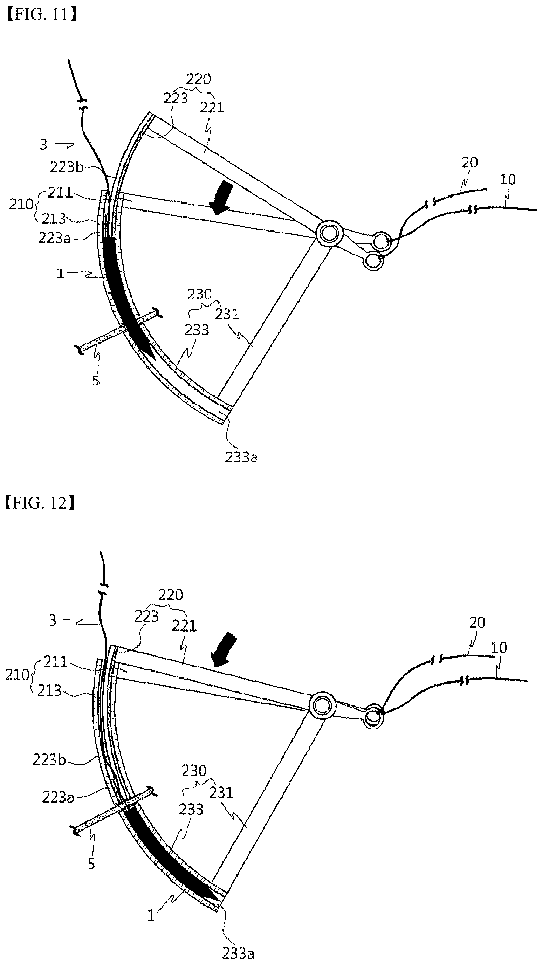

FIGS. 11 and 12 are views for explaining suturing of a suture spot according to a second operation of the surgical suture instrument of the present invention.

First, as shown in FIG. 11, when the second operation unit 330 of the driver 300 is pulled by a finger, the second operation unit 330 is compressed and the second wire 20 pulls the push link member 221, and thus, the push guide member 223 is rotated toward the inlet guide 210 about the first linkage 110. Then, the push guide member 223 is inserted into the inlet guide member 213 to rotate and push the suture needle 1 in the inlet guide member 213 so that the suture needle 1 passes through the suture spot 5. Here, the leading end of the suture needle 1 passing through the suture spot 5 is introduced into the outlet hole 233a of the outlet guide member 233.

Next, as shown in FIG. 12, when the push guide member 223 is further rotated to substantially coincide with the inlet guide member 213 to fully push the suture needle 1 inserted into the inlet guide member 213, the entire suture needle 1 passes through the suture spot 5 so that the suture wire 3 connected to the suture needle 1 sutures the suture spot 5. Here, the suture needle 1 completely passed through the suture spot 5 is guided to pass through the outlet hole 233a of the outlet guide member 233.

Next, compression against the first and second operation units 320 and 330 is released and the surgical suture instrument is separated from the suture spot 5, completing a single suture with respect to the suture spot 5 of the tissue.

Finally, after the completion of the single suture, the suture needle 1 is inserted into the inlet guide 210 again, and the process of FIGS. 9 to 12 is repeated to perform the entire suturing with respect to the suture spot 5 of the tissue.

Accordingly, the surgical suture instrument of the present invention squeezes the desired suture spot 5 through a primary rotation after insertion of the curved suture needle 1, and pushes the suture needle 1 to pass through the suture spot 5 by a secondary rotation to enable an elaborate suture with respect to the suture spot 5, minimizing undesired damage to the tissue by the suture needle 1. In addition, as the squeezing and suturing operation of the suture spot 5 is performed by the rotation, the suturing can be safely performed regardless of the direction of the suture plane. Further, since manipulation of the suture needle 1 simple, an operation time can be reduced. Furthermore, since the suturing using a single laparoscopic port can be performed, an incision surface of the body can be minimized.

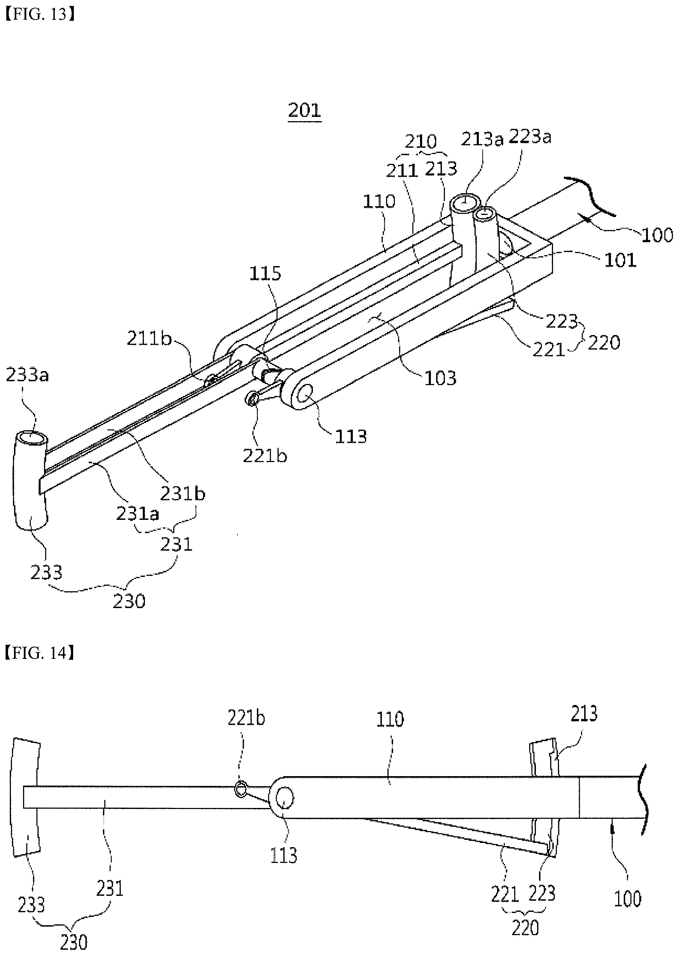

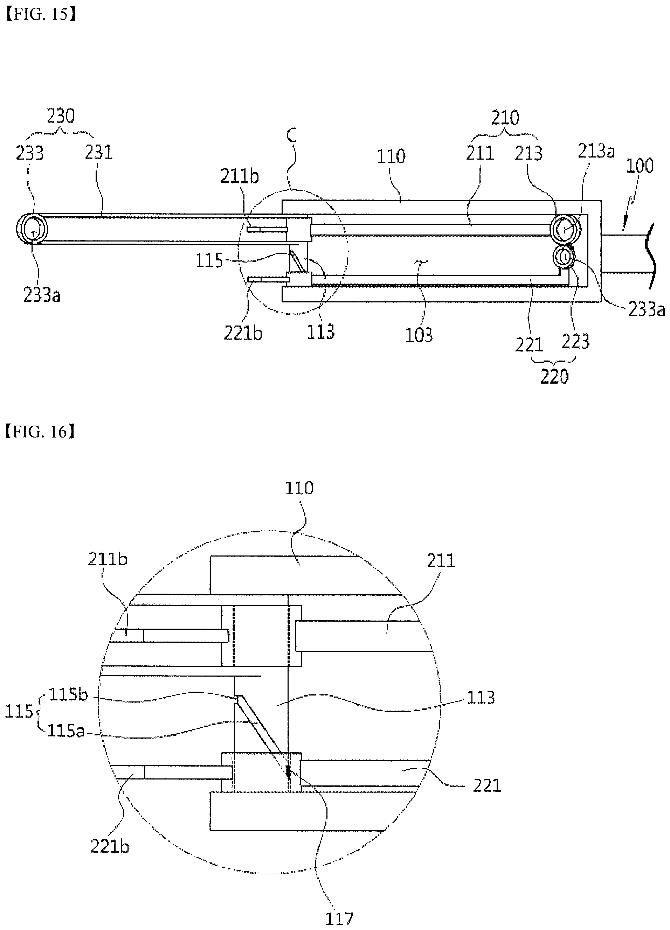

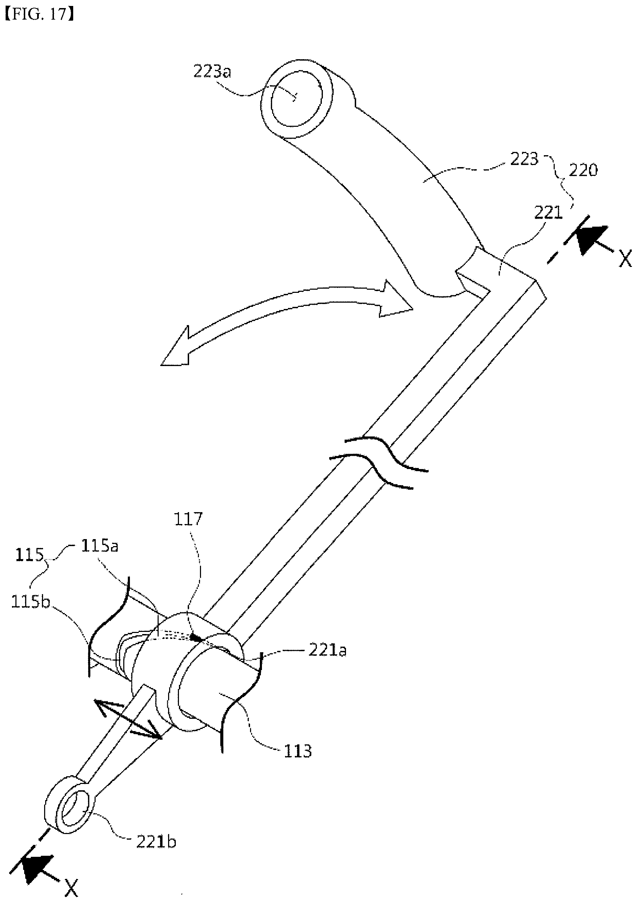

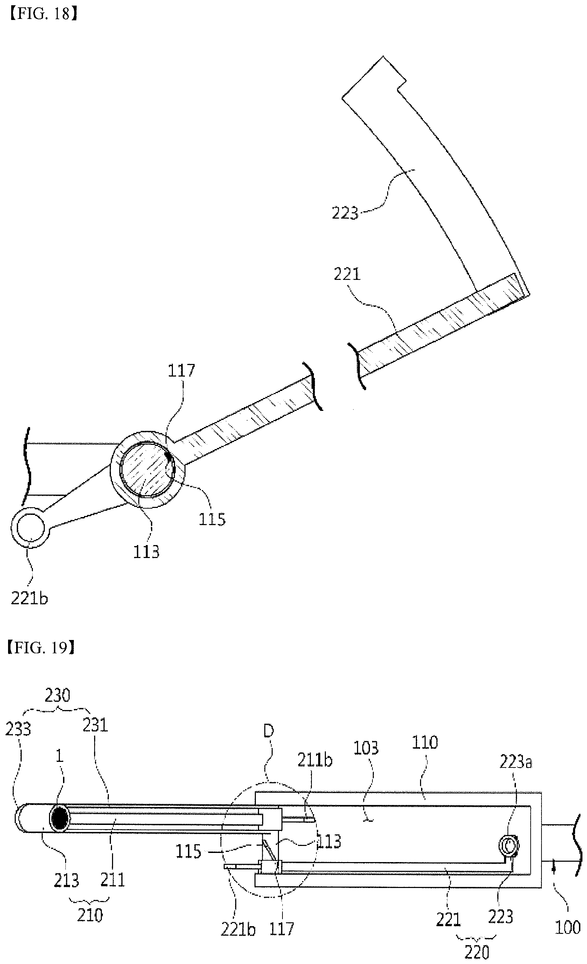

FIG. 13 is a perspective view of a needle actuator in accordance with another exemplary embodiment of the present invention, FIG. 14 is a side view of FIG. 13, FIG. 15 is a plan view of FIG. 13, FIG. 16 is an enlarged view of part C shown in FIG. 15, FIG. 17 is a perspective view showing a rotational moving structure of a needle pusher with respect to a fixing pin, and FIG. 18 is a cross-sectional view taken along line X-X of FIG. 17.

As shown in FIGS. 13 to 18, a needle actuator 201 in accordance with another exemplary embodiment of the present invention may include an inlet guide 210, a needle pusher 220 and an outlet guide 230.

The needle actuator 201 of this embodiment is similar to the needle actuator 200 according to the previous embodiment of the present invention shown in FIG. 4 except for a rotation and movement structure of a needle pusher 220 with respect to a fixing pin 113. Accordingly, the same components as in the previous embodiment will be omitted.

The inlet guide 210 is rotatably coupled to one side of a leading end of a shank 100, is connected to the first wire 10 (see FIG. 6), and functions to guide such that the suture needle 1 is inserted.

The inlet guide 210 may include an inlet link member 211 having a link connecting hole 211a (see FIG. 5) fitted onto the fixing pin 113 fixed into the leading end of the first linkage 110 of the shank 100 and coupled to rotate toward the outlet guide 230 about the fixing pin 113, and an inlet guide member 213 fixed to an end of the inlet link member 211 and having an inlet hole 213a into which the suture needle 1 is inserted. Here, since the link connecting hole 211a of the inlet link member 211 is disposed between a pair of outlet fixture members 231a and 231b, there is no movement with respect to the fixing pin 113 in a direction of a rotary shaft when the inlet guide 210 is rotated.

In addition, a first wire connecting hole 211b (see FIG. 5), into which the first wire 10 is connected to an opposite area of the inlet guide member 213 with respect to the link connecting hole 211a connected to the fixing pin 113, may be formed in the inlet link member 211.

The inlet guide member 213 has an annular cross-section, and may have an arc shape to correspond to the curved suture needle 1 when seen from a side view.

Further, the inlet guide member 213 is positioned in the long hole 103 of the first linkage 110 at an initial stage to minimize an outer cross-sectional area, preventing damage to internal organs of the body when the needle actuator 201 approaches an operation area of a patient.

The needle pusher 220 is rotatably connected to the other side of the leading end of the shank 100, is connected to the second wire 20 (see FIG. 6), and functions to push the suture needle 1 such that the suture needle 1 inserted into the inlet guide 210 passes through the suture spot 5 to perform the suturing.

The needle pusher 220 may include a push link member 221 coupled such that the link connecting hole 221a (see FIG. 5) is fitted onto the fixing pin 113 inserted into the leading end of the first linkage 110 to subsequently rotate toward the inlet guide 210 previously rotated toward the outlet guide 230, and a push guide member 223 fixed to an end of the push link member 221 and rotated and inserted into the inlet guide member 213 to push the suture needle 1 inserted into the inlet guide member 213.

Here, a movable guide groove 115 including an inclined guide groove 115a inclined toward the link connecting hole 211a of inlet link member 211 at a position of the link connecting hole 221a of the push link member 221 and a straight guide groove 115b straightly formed at an end of a front inclination of the inclined guide groove 115a in a circumferential direction of the fixing pin 113 is provided at an outer circumference of the fixing pin 113, and a movable guide protrusion 117 is formed in the link connecting hole 221a of the push link member 221 to be inserted into the movable guide groove 115. This is provided to guide the push guide member 223 to coincide with a radius of rotation of the inlet guide member 213 as the movable guide protrusion 117 moves along the movable guide groove 115 when the needle pusher 220 is subsequently rotated toward the inlet guide 210 previously rotated about the fixing pin 113.

In addition, the push link member 221 may have a second wire connecting hole 221b (see FIG. 5) to which the second wire 20 is connected at an opposite area of the push guide member 223 with respect to the link connecting hole 221a connected to the fixing pin 113.

The push guide member 223 has an arc shape corresponding to the inlet guide member 213 to be inserted into the arc-shaped inlet guide member 213.

Further, the push guide member 223 is positioned in the long hole 103 of the first linkage 110 to be in parallel with the inlet guide member 213 at an initial stage to minimize an outer cross-sectional area, preventing damage to internal organs of the body, when the needle actuator 201 approaches an operation area of the patient.

The outlet guide 230 is fixed to the leading end of the shank 100, is engaged with the inlet guide 210 to squeeze the suture spot 5, and functions to guide passage of the suture needle 1 passing through the suture spot 5.

The outlet guide 230 may include an outlet fixture member 231 fixed to the fixing pin 113 provided at the leading end of the first linkage 110 or the leading end of the first linkage 110 to be disposed in front of the shank 100 on a straight line, and an outlet guide member 233 fixed to an end of the outlet fixture member 231 and having an outlet hole 233a through which the suture needle 1 passing through the suture spot 5 passes.

In addition, the inlet guide member 213, the push guide member 223 and the outlet guide member 233 may be circumferentially disposed along a concentric circle having the same radius about the fixing pin 113 of the first linkage 110.

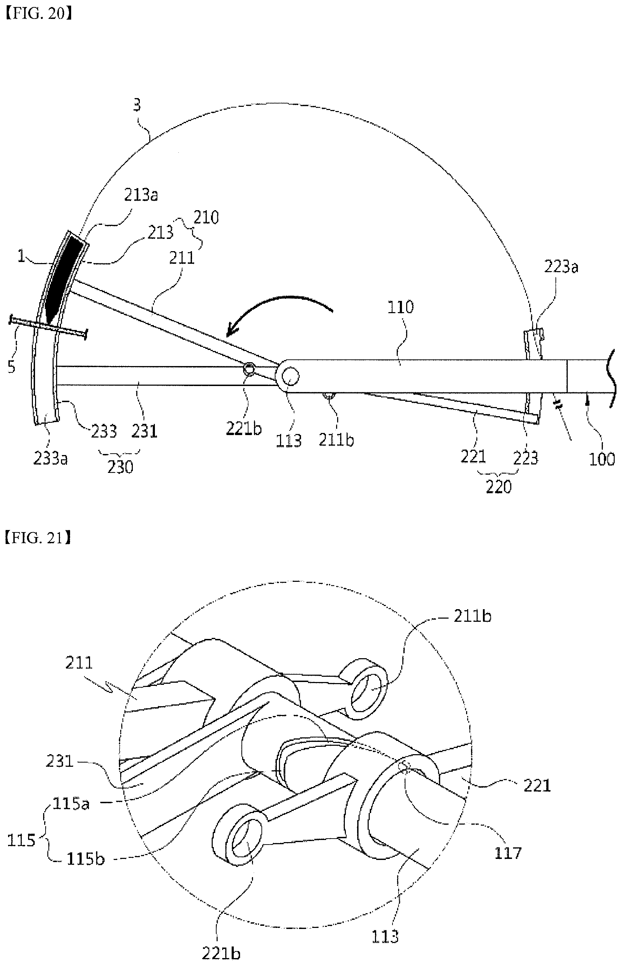

FIG. 19 is an example view for explaining a first operation state of the needle actuator in accordance with another exemplary embodiment of the present invention, FIG. 20 is a plan view of FIG. 19, and FIG. 21 is an enlarged view of part D shown in FIG. 20.

As shown in FIGS. 19 to 21, in a state in which the suture needle 1 is inserted into the inlet guide member 213, the desired suture spot 5 of the tissue is positioned between the inlet guide member 213 and the outlet guide member 233. Then, when the first operation unit 320 of the driver 300 is pulled by a finger, the first operation unit 320 is compressed, and the first wire 10 pulls the inlet link member 211. Then, the inlet guide member 213 into which the suture needle 1 is inserted is rotated toward the outlet guide 230 about the fixing pin 113 of the first linkage 110, and the inlet guide member 213 is engaged with the outlet guide member 233 to squeeze the suture spot 5. Here, the needle pusher 220 is maintained at an initial stop state in the long hole 103 of the first linkage 110. In addition, the movable guide protrusion 117 in the link connecting hole 221a of the push link member 221 is maintained in a state inserted into a rear side position of the inclined guide groove 115a formed in the outer circumference of the fixing pin 113.

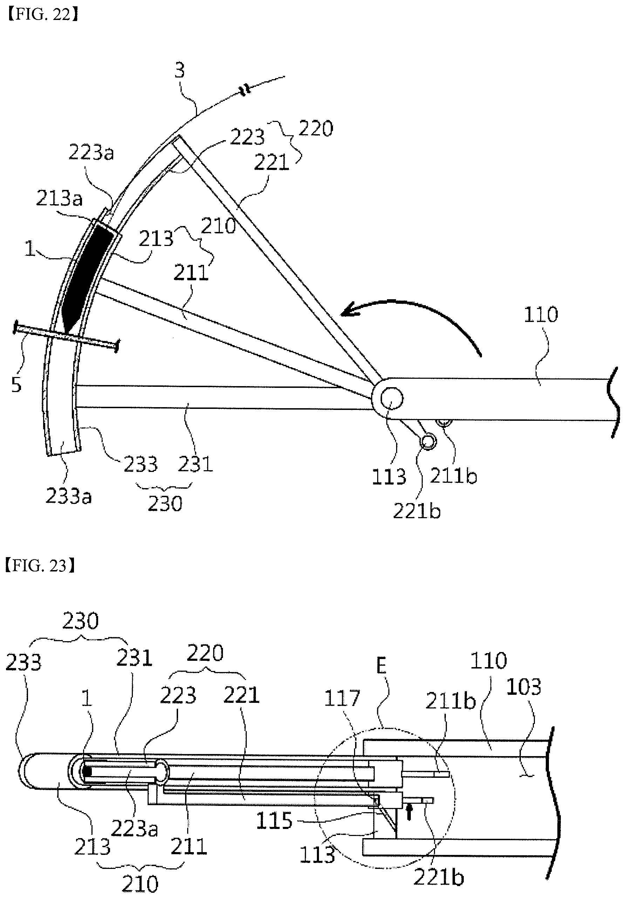

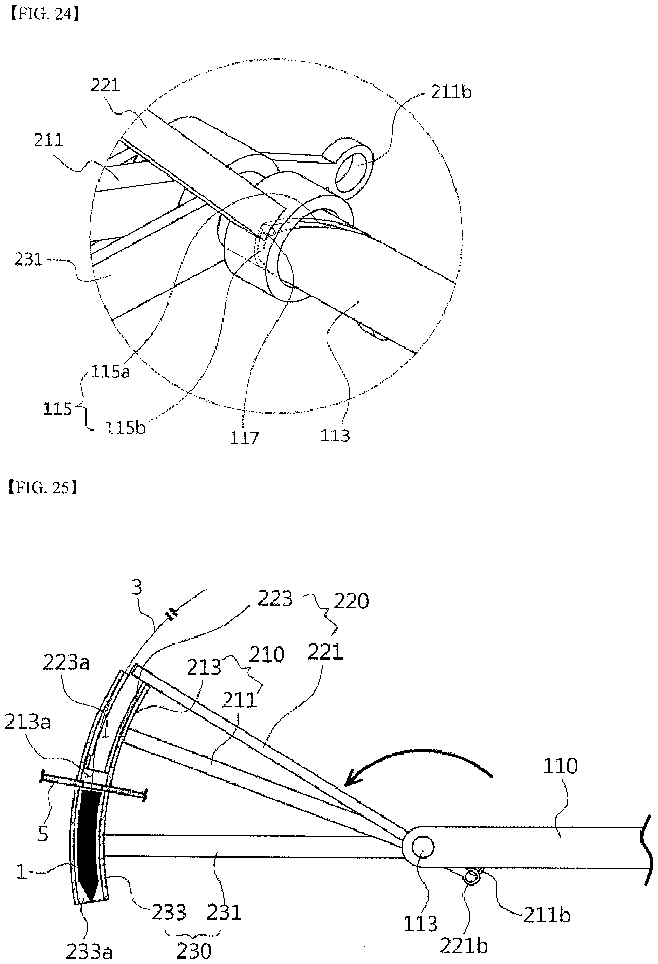

FIG. 22 is an example view for explaining a second operation state of the needle actuator, FIG. 23 is a plan view of FIG. 22, and FIG. 24 is an enlarged view of part E shown in FIG. 23.

As shown in FIGS. 22 to 24, when the second operation unit 330 of the driver 300 is pulled by a finger, the second operation unit 330 is compressed and the second wire 20 pulls the push link member 221, and the push guide member 223 is rotated toward the inlet guide 210 about the fixing pin 113 of the first linkage 110. Here, the movable guide protrusion 117 in the link connecting hole 221a of the push link member 221 is moved just before the straight guide groove 115b along the inclined guide groove 115a of the movable guide groove 115, i.e., to a boundary area between the inclined guide groove 115a and the straight guide groove 115b. Accordingly, the push guide member 223 is moved toward the inlet guide member 213 in the axial direction of the fixing pin 113 such that the leading end of the push guide member 223 is positioned near the trailing end of the inlet guide member 213.

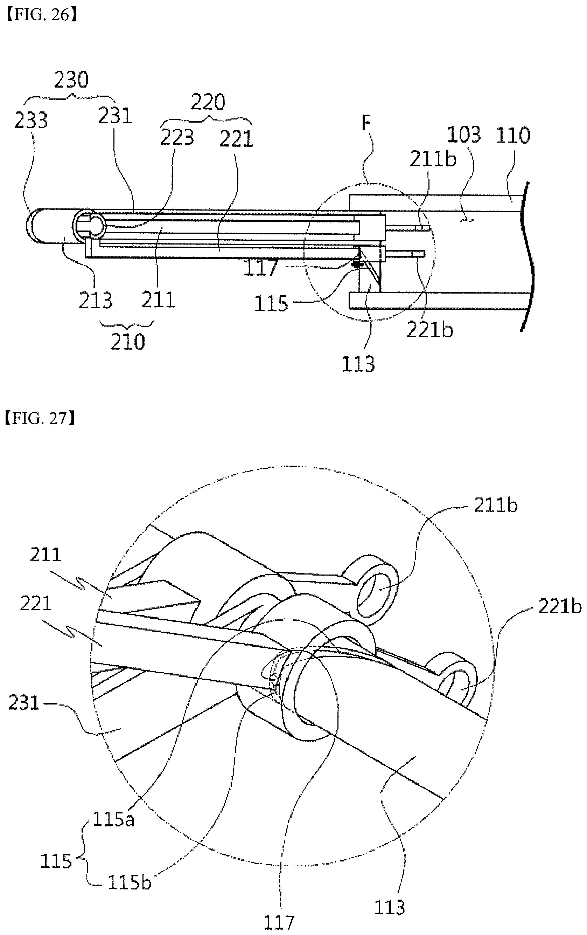

FIG. 25 is an example for explaining a third operation state of the needle actuator, FIG. 26 is a plan view of FIG. 25, and FIG. 27 is an enlarged view of part F shown in FIG. 26.

As shown in FIGS. 25 to 27, when the push guide member 223 is further rotated into the inlet guide member 213, the movable guide protrusion 117 in the link connecting hole 221a of the push link member 221 further moves forward along the straight guide groove 115b of the movable guide groove 115, and the push guide member 223 pushes the suture needle 1 inserted into the inlet guide member 213 to pass through the suture spot 5. Then, the entire suture needle 1 passes through the suture spot 5, and the suture wire 3 connected to the suture needle 1 passes through the suture spot 5. Here, the suture needle 1 completely passed through the suture spot 5 is guided to pass through the outlet hole 233a of the outlet guide member 233.

Next, compression against the first and second operation units 320 and 330 is released and the surgical suture instrument is separated from the suture spot 5, completing a single suture with respect to the suture spot 5 of the tissue.

It will be understood to those skilled in the art that the present invention may be implemented in various ways without changing the spirit of necessary features of the present invention. Accordingly, the embodiments described above are provided as examples in the whole respects and do not limit the present invention. The scope of the present invention is defined in the following claims and all changed or modified types derived from the meanings and scope of the claims and the equivalent concept thereof should be construed as being included in the scope of the present invention.

### [Industrial Applicability]

The present invention can be applied to a surgical suture instrument in which elaborate and safe suturing is needed, while minimizing undesired damage to the tissue by the suture needle in the event of a laparotomy and laparoscopic surgery.

## Claims

1. A surgical suture instrument comprising:
a shank (100);
a needle actuator (200) installed at one side of the shank (100), into which a suture needle (1) is inserted, squeezing a desired suture spot through primary rotation, and pushing the suture needle (1) by secondary rotation to pass through the suture spot to suture the suture spot; and
a driver (300) installed at the other side of the shank (100) and sequentially driving the squeezing and suturing operations of the needle actuator (200), wherein the shank (100) has a wire hole (101) in which first and second wires (10, 20) are held, and
the needle actuator (200) comprises:
an inlet guide (210) rotatably coupled to one side of a leading end of the shank (100), connected to the first wire (10), and guiding insertion of the suture needle (1);
a needle pusher (220) rotatably coupled to the other side of the leading end of the shank (100), connected to the second wire (20), and rotating toward the inlet guide (210) to push the suture needle (1) such that the suture needle (1) inserted into the inlet guide (210) passes through the suture spot to suture the suture spot; and
an outlet guide (230) fixed to the leading end of the shank (100), engaging with the inlet guide (210) to squeeze the suture spot, and guiding passage of the suture needle (1) passing through the suture spot.

2. The surgical suture instrument according to claim 1, wherein the inlet guide (210) and the needle pusher (220) are rotatably connected to the leading end of the shank (100), and a first linkage (110) to which the outlet guide (230) is fixedly connected is provided at the leading end.

3. The surgical suture instrument according to claim 2, wherein a long hole (103) corresponding to a radius of rotation of the needle pusher (220) is formed in the first linkage (110) such that the needle pusher (220) is rotated toward the inlet guide (210) to pass therethrough.

4. The surgical suture instrument according to claim 2, wherein the inlet guide (210) comprises:
an inlet link member (211) rotatably linked to the first linkage (110) about an axis perpendicular to a longitudinal direction of the shank (100); and
an inlet guide member (213) fixed to the inlet link member (211), having an arc shape corresponding to the curved suture needle (1), and having an inlet hole (213a) into which the suture needle (1) is inserted.

5. The surgical suture instrument according to claim 4, wherein the inlet link member (211) has a first wire connecting hole (211b), to which the first wire (10) is connected, formed at an opposite area of the inlet guide member (213) with respect to the first linkage (110).

6. The surgical suture instrument according to claim 4, wherein the needle pusher (220) comprises:
a push link member (221) rotatably linked to the first linkage (110) about the same axis as the inlet link member (211); and
a push guide member (223) fixed to the push link member (221), having an arc shape corresponding to the inlet guide member (213), and rotated to be inserted into the inlet guide member (213) and pushing the suture needle (1) inserted into the inlet guide member (213).

7. The surgical suture instrument according to claim 6, wherein the push link member (221) has a second wire connecting hole (221b), to which the second wire (20) is connected, formed at an opposite area of the push guide member (223) with respect to the first linkage (110).

8. The surgical suture instrument according to claim 6, wherein the outlet guide (230) comprises:
an outlet fixture member (231) fixed to the first linkage (110) by a fixing shaft (232) having an angle perpendicular to a longitudinal direction of the shank (100); and
an outlet guide member (233) fixed to the outlet fixture member (231), having an arc shape corresponding to the inlet guide member (213), and having an outlet hole (233a) through which the suture needle (1) passing through the suture spot passes.

9. The surgical suture instrument according to claim 8, wherein the inlet guide member (213), the push guide member (223) and the outlet guide member (233) are circumferentially disposed along a concentric circle having the same radius about the first linkage (110).

10. The surgical suture instrument according to claim 1, wherein the driver (300) is installed at a trailing end of the shank (100), and provides tension to the first wire (10) and the second wire (20) to sequentially rotate the inlet guide (210) and the needle pusher (220).

11. The surgical suture instrument according to claim 10, wherein the driver (300) comprises:
a handle (300) coupled to the trailing end of the shank (100) and gripped by a user;
a first operation unit (320) pivotally coupled to one side of the handle (300), connected to the first wire (10), and providing tension to the first wire (10) by compression of the user to rotate the inlet guide (210) toward the outlet guide (230); and
a second operation unit (330) pivotally coupled to the other side of the handle (300), connected to the second wire (20), and providing tension to the second wire (20) by compression of the user to rotate the needle pusher (220) toward the inlet guide (210).

12. The surgical suture instrument according to claim 11, wherein a second linkage (120) to which the handle (300) is rotatably connected is provided at the trailing end of the shank (100).

## Patentansprüche

1. Ein chirurgisches Nahtinstrument, das folgende Merkmale aufweist:
einen Schaft (100);
ein Nadelbetätigungsbauglied (200), das auf einer Seite des Schafts (100) installiert ist und in das eine Nahtnadel (1) eingeführt wird, wobei ein gewünschter Nahtpunkt durch primäre Drehung zusammengedrückt wird, und Schieben der Nahtnadel (1) anhand einer sekundären Drehung, damit sie durch den Nahtpunkt gelangt, um den Nahtpunkt zu nähen; und
einen Treiber (300), der auf der anderen Seite des Schafts (100) installiert ist und den Zusammendrück- und den Nähvorgang des Nadelbetätigungsgliedes (200) nacheinander treibt,
wobei der Schaft (100) ein Drahtloch (101) aufweist, in dem ein erster und ein zweiter Draht (10, 20) gehalten werden, und
das Nadelbetätigungsglied (200) folgende Merkmale aufweist:
eine Einlassführung (210), die drehbar mit einer Seite eines Vorderendes des Schafts (100) gekoppelt ist, mit dem ersten Draht (10) verbunden ist und eine Einführung der Nahtnadel (1) führt;
einen Nadelschieber (220), der drehbar mit der anderen Seite des Vorderendes des Schafts (100) gekoppelt ist, mit dem zweiten Draht (20) verbunden ist und sich zu der Einlassführung (210) hin dreht, um die Nahtnadel (1) derart zu schieben, dass die in die Einlassführung (210) eingeführte Nahtnadel (1) durch den Nahtpunkt gelangt, um den Nahtpunkt zu nähen; und
eine Auslassführung (230), die an dem Vorderende des Schafts (100) befestigt ist, mit der Einlassführung (210) in Eingriff gelangt, um den Nahtpunkt zusammenzudrücken, und ein Hindurchgelangen der durch den Nahtpunkt gelangenden Nahtnadel (1) führt.

2. Das chirurgische Nahtinstrument gemäß Anspruch 1, bei dem die Einlassführung (210) und der Nadelschieber (220) drehbar mit dem Vorderende des Schafts (100) verbunden sind und ein erstes Verbindungsglied (110), mit dem die Auslassführung (230) fest verbunden ist, an dem Vorderende vorgesehen ist.

3. Das chirurgische Nahtinstrument gemäß Anspruch 2, bei dem ein Langloch (103), das einem Drehradius des Nadelschiebers (220) entspricht, derart in dem ersten Verbindungsglied (110) gebildet ist, dass der Nadelschieber (220) zu der Einlassführung (210) hin gedreht wird, um durch dieselbe hindurch zu gelangen.

4. Das chirurgische Nahtinstrument gemäß Anspruch 2, bei dem die Einlassführung (210) folgende Merkmale aufweist:
ein Einlassverbindungsbauglied (211), das um eine Achse, die senkrecht zu einer Längsrichtung des Schafts (100) ist, herum drehbar mit dem ersten Verbindungsglied (110) verbunden ist; und
ein Einlassführungsbauglied (213), das an dem Einlassverbindungsbauglied (211) befestigt ist, eine Bogenform aufweist, die der gekrümmten Nahtnadel (1) entspricht, und ein Einlassloch (213a) aufweist, in das die Nahtnadel (1) eingeführt wird.

5. Das chirurgische Nahtinstrument gemäß Anspruch 4, bei dem das Einlassverbindungsbauglied (211) ein erstes Drahtverbindungsloch (211b), mit dem der erste Draht (10) verbunden ist, aufweist, das an einem gegenüberliegenden Bereich des Einlassführungsbauglieds (213) bezüglich des ersten Verbindungsglieds (110) gebildet ist.

6. Das chirurgische Nahtinstrument gemäß Anspruch 4, bei dem der Nadelschieber (220) folgende Merkmale aufweist:
ein Schiebeverbindungsbauglied (221), das um dieselbe Achse wie das Einlassverbindungsbauglied (211) herum drehbar mit dem ersten Verbindungsglied (110) verbunden ist; und
ein Schiebeführungsbauglied (223), das an dem Schiebeverbindungsbauglied (221) befestigt ist, das eine Bogenform aufweist, die dem Einlassführungsbauglied (213) entspricht, und das gedreht wird, um in das Einlassführungsbauglied (213) eingeführt zu werden, und das die in das Einlassführungsbauglied (213) eingeführte Nahtnadel (1) schiebt.

7. Das chirurgische Nahtinstrument gemäß Anspruch 6, bei dem das Schiebeverbindungsbauglied (221) ein zweites Drahtverbindungsloch (221b) aufweist, mit dem der zweite Draht (20) verbunden ist und das an einem gegenüberliegenden Bereich des Schiebeführungsbauglieds (223) bezüglich des ersten Verbindungsglieds (110) gebildet ist.

8. Das chirurgische Nahtinstrument gemäß Anspruch 6, bei dem die Auslassführung (230) folgende Merkmale aufweist:
ein Auslassbefestigungsbauglied (231), das anhand einer Befestigungswelle (232), die einen zu einer Längsrichtung des Schafts (100) senkrechten Winkel aufweist, an dem ersten Verbindungsglied (110) befestigt ist; und
ein Auslassführungsbauglied (233), das an dem Auslassbefestigungsbauglied (231) befestigt ist, eine Bogenform aufweist, die dem Einlassführungsbauglied (213) entspricht, und ein Auslassloch (233a) aufweist, durch das die Nahtnadel (1) gelangt, die durch den Nahtpunkt gelangt.

9. Das chirurgische Nahtinstrument gemäß Anspruch 8, bei dem das Einlassführungsbauglied (213), das Schiebeführungsbauglied (223) und das Auslassführungsbauglied (233) umfangsmäßig entlang eines konzentrischen Kreises angeordnet sind, der um das erste Verbindungsglied (110) herum denselben Radius aufweist.

10. Das chirurgische Nahtinstrument gemäß Anspruch 1, bei dem der Treiber (300) an einem hinteren Ende des Schafts (100) installiert ist und dem ersten Draht (10) und dem zweiten Draht (20) eine Spannung bereitstellt, um die Einlassführung (210) und den Nadelschieber (220) nacheinander zu drehen.

11. Das chirurgische Nahtinstrument gemäß Anspruch 10, bei dem der Treiber (300) folgende Merkmale aufweist:
einen Griff (300), der mit dem hinteren Ende des Schafts (100) gekoppelt ist und seitens eines Nutzers gegriffen wird;
eine erste Bedieneinheit (320), die schwenkbar mit einer Seite des Griffs (300) gekoppelt ist, mit dem ersten Draht (10) verbunden ist und dem ersten Draht (10) durch Komprimieren seitens des Nutzers eine Spannung bereitstellt, um die Einlassführung (210) zu der Auslassführung (230) hin zu drehen; und
eine zweite Bedieneinheit (330), die schwenkbar mit der anderen Seite des Griffs (300) gekoppelt ist, mit dem zweiten Draht (20) verbunden ist und dem zweiten Draht (20) durch Komprimieren seitens des Nutzers eine Spannung bereitstellt, um den Nadelschieber (220) zu der Einlassführung (210) hin zu drehen.

12. Das chirurgische Nahtinstrument gemäß Anspruch 11, bei dem ein zweites Verbindungsglied (120), mit dem der Griff (300) drehbar verbunden ist, an dem hinteren Ende des Schafts (100) vorgesehen ist.

## Revendications

1. Instrument à sutures chirurgicales, comprenant:
un manche (100);
un actionneur à aiguille (200) installé d'un côté du manche (100), dans lequel est introduite une aiguille de suture (1), comprimant un point de suture souhaité par rotation primaire et poussant l'aiguille de suture (1) par rotation secondaire de manière à passer à travers le point de suture pour suturer le point de suture; et
un dispositif de commande (300) installé de l'autre côté du manche (100) et commandant en séquence les opérations de compression et de suture de l'actionneur à aiguille (200),
dans lequel le manche (100) présente un trou à fils (101) dans lequel sont maintenus les premier et deuxième fils (10, 20), et
l'actionneur à aiguille (200) comprend:
un guide d'entrée (210) couplé de manière rotative à un côté d'une extrémité avant du manche (100), connecté au premier fil (10) et guidant l'introduction de l'aiguille de suture (1);
un poussoir d'aiguille (220) couplé de manière rotative à l'autre côté de l'extrémité avant du manche (100), connecté au deuxième fil (20) et tournant vers le guide d'entrée (210) pour pousser l'aiguille de suture (1) de sorte que l'aiguille de suture (1) introduite dans le guide d'entrée (210) passe à travers le point de suture pour suturer le point de suture; et
un guide de sortie (230) fixé à l'extrémité avant du manche (100), venant en prise avec le guide d'entrée (210) pour comprimer le point de suture, et guidant le passage de l'aiguille de suture (1) passant à travers le point de suture.

2. Instrument à sutures chirurgicales selon la revendication 1, dans lequel le guide d'entrée (210) et le poussoir d'aiguille (220) sont connectés de manière rotative à l'extrémité avant du manche (100) et une première tringlerie (110), à laquelle est connecté de manière fixe le guide de sortie (230), est prévue à l'extrémité avant.

3. Instrument à sutures chirurgicales selon la revendication 2, dans lequel un trou long (103) correspondant à un rayon de rotation du poussoir d'aiguille (220) est formé dans la première tringlerie (110) de sorte que le poussoir d'aiguille (220) soit tourné vers le guide d'entrée (210) pour passer à travers ce dernier.

4. Instrument à sutures chirurgicales selon la revendication 2, dans lequel le guide d'entrée (210) comprend:
un élément de liaison d'entrée (211) connecté de manière rotative à la première tringlerie (110) autour d'un axe perpendiculaire à une direction longitudinale du manche (100); et
un élément de guidage d'entrée (213) fixé à l'élément de liaison d'entrée (211), présentant une forme en arc correspondant à l'aiguille de suture courbée (1) et présentant un orifice d'entrée (213a) dans lequel est introduite l'aiguille de suture (1).

5. Instrument à sutures chirurgicales selon la revendication 4, dans lequel l'élément de liaison d'entrée (211) présente un premier trou de connexion de fil (211b) auquel est connecté le premier fil (10), formé dans une zone opposée de l'élément de guidage d'entrée (213) par rapport à la première tringlerie (110).

6. Instrument à sutures chirurgicales selon la revendication 4, dans lequel le poussoir d'aiguille (220) comprend:
un élément de liaison de poussée (221) connecté de manière rotative à la première tringlerie (110) autour du même axe que l'élément de liaison d'entrée (211); et
un élément de guidage de poussée (223) fixé à l'élément de liaison de poussée (221), présentant une forme en arc correspondant à l'élément de guidage d'entrée (213), et tourné de manière à être introduit dans l'élément de guidage d'entrée (213) et poussant l'aiguille de suture (1) introduite dans l'élément de guidage d'entrée (213).

7. Instrument à sutures chirurgicales selon la revendication 6, dans lequel l'élément de liaison de poussée (221) présente un deuxième trou de connexion de fil (221b) auquel est connecté le deuxième fil (20), formé dans une zone opposée de l'élément de guidage de poussée (223) par rapport à la première tringlerie (110).

8. Instrument à sutures chirurgicales selon la revendication 6, dans lequel le guide de sortie (230) comprend:
un élément de fixation de sortie (231) fixé à la première tringlerie (110) par un arbre de fixation (232) présentant un angle perpendiculaire à une direction longitudinale du manche (100); et
un élément de guidage de sortie (233) fixé à l'élément de fixation de sortie (231), présentant une forme en arc correspondant à l'élément de guidage d'entrée (213) et présentant un trou de sortie (233a) à travers lequel passe l'aiguille de suture (1) passant à travers le point de suture.

9. Instrument à sutures chirurgicales selon la revendication 8, dans lequel l'élément de guidage d'entrée (213), l'élément de guidage de poussée (223) et l'élément de guidage de sortie (233) sont disposés de manière circonférentielle le long d'un cercle concentrique présentant le même rayon autour de la première tringlerie (110).

10. Instrument à sutures chirurgicales selon la revendication 1, dans lequel le dispositif de commande (300) est installé à une extrémité arrière du manche (100) et fournit une tension au premier fil (10) et au deuxième fil (20) pour faire tourner en séquence le guide d'entrée (210) et le poussoir d'aiguille (220).

11. Instrument à sutures chirurgicales selon la revendication 10, dans lequel le dispositif de commande (300) comprend:
une poignée (300) couplée à l'extrémité arrière du manche (100) et saisie par un utilisateur;
une première unité d'actionnement (320) couplée de manière pivotante à un côté de la poignée (300), connectée au premier fil (10), et fournissant une tension au premier fil (10) par compression par l'utilisateur pour faire tourner le guide d'entrée (210) vers le guide de sortie (230); et
une deuxième unité d'actionnement (330) couplée de manière pivotante à l'autre côté de la poignée (300), connectée au deuxième fil (20) et fournissant une tension au deuxième fil (20) par compression par l'utilisateur pour faire tourner le poussoir d'aiguille (220) vers le guide d'entrée (210).

12. Instrument à sutures chirurgicales selon la revendication 11, dans lequel une deuxième tringlerie (120), à laquelle est connectée de manière rotative la poignée (300), est prévue à l'extrémité arrière du manche (100).
